(19) Europäisches Patentamt
European Patent Office
Office européen des brevets

(11) Numéro de publication : **0 072 293**
**B1**

(12) # FASCICULE DE BREVET EUROPÉEN

(45) Date de publication du fascicule du brevet : **09.10.85**

(51) Int. Cl.[4] : **C 07 J 9/00**

(21) Numéro de dépôt : **82401408.8**

(22) Date de dépôt : **28.07.82**

(54) **Procédé de préparation de l'acide ursodésoxycholique à partir de l'acide 3 alpha, 7 beta, 12 alpha-trihydroxycholanique et produits intermédiaires utilisés.**

(30) Priorité : **07.08.81 FR 8115347**

(43) Date de publication de la demande : **16.02.83 Bulletin 83/07**

(45) Mention de la délivrance du brevet : **09.10.85 Bulletin 85/41**

(84) Etats contractants désignés : **AT BE CH DE GB IT LI NL**

(56) Documents cités :
**EP-A- 0 063 106**
**CHEMICAL ABSTRACTS, vol. 82, no. 19, 12 mai 1975, page 576, no. 125527b, Columbus Ohio (USA); CHEMICAL ABSTRACTS, vol. 92, no. 7, 18 fevrier 1980, page 709 no. 59113s, Columbus Ohio (USA); CHEMICAL ABSTRACTS, vol. 51, no. 22, 25 novembre 1957, col. 17965b-c, Columbus Ohio (USA); T. KANAZAWA et al.: "Synthesis of ursodeoxycholic acid"**

(73) Titulaire : **ROUSSEL-UCLAF**
**35, boulevard des Invalides**
**F-75007 Paris (FR)**

(72) Inventeur : **Bulidon, Jacques**
**Place du Monument aux Morts**
**F-86350-Usson du Poitou (FR)**
Inventeur : **Demuynck, Robert**
**92, rue de la Fédération**
**F-93100-Montreuil Sous Bois (FR)**
Inventeur : **Pavan, Charles**
**26-28, Grande Rue Charles De Gaulle**
**F-94130-Nogent Sur Marne (FR)**

(74) Mandataire : **Bourgouin, André et al**
**ROUSSEL-UCLAF 111, route de Noisy Boîte postale no 9**
**F-93230 Romainville (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 072 293 B1**

Jouve, 18, rue St-Denis, 75001 Paris, France

**Description**

La présente demande concerne un nouveau procédé de préparation de l'acide ursodésoxycholique ou acide 3α, 7β-dihydroxycholanique.

De nombreuses synthèses de cet acide ont été décrites qui consistent à réduire sélectivement l'acide 3α-hydroxy 7-céto cholanique. On peut citer, par exemple, les synthèses décrites par KANAZAWA et al. CA, 51 (1957) 17965, dans les demandes de brevets japonais n° 154865/1975 et 82722/1975 ainsi que dans le brevet français 2.444.048.

La présente demande décrit une synthèse en quatre stades à partir de l'acide 3α, 7β, 12α-trihydroxycholanique.

Ce produit est décrit, par exemple, dans la publication Acta Technica Scandinavica 14, 1960, pages 17-20.

La présente synthèse est caractérisée en ce que l'on traite l'acide 3α, 7/3, 12α-trihydroxycholanique de formule II :

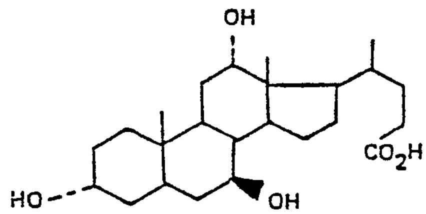

(II)

par un dérivé réactionnel d'un radical organique de formule :

R—CO—

dans laquelle R représente un atome d'hydrogène ou un radical alkyle ou alkoxy ayant de 1 à 5 atomes de carbone ou alkényloxy ayant de 2 à 5 atomes de carbone, un radical cycloalkyloxy ayant de 3 à 7 atomes de carbone ou aralkyloxy ayant de 7 à 12 atomes de carbone, un radical aryle ou aralkyle, ayant de 6 à 12 atomes de carbone, chacun de ces radicaux portant éventuellement un ou plusieurs substituants, pour obtenir un produit de formule III :

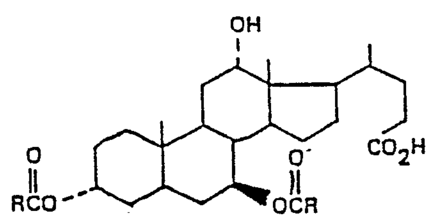

(III)

produit de formule III que l'on traite par un agent d'oxydation pour obtenir un produit de formule IV :

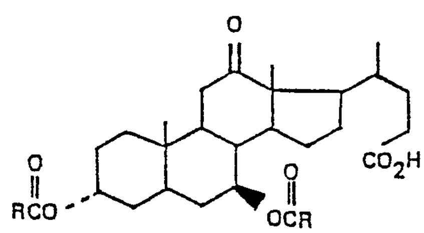

(IV)

produit de formule IV que l'on saponifie pour obtenir un produit de formule V :

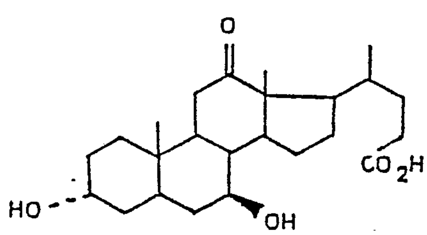

(V)

où un sel de ce produit, produit de formule V ou sel que l'on traite par un agent de réduction pour obtenir l'acide ursodésoxycholique cherché.

Le dérivé réactionnel d'un radical organique que l'on peut utiliser pour traiter l'acide 3α, 7β, 12α-trihydroxycholanique peut être choisi parmi l'acide formique et les dérivés des acides acétique, propionique, butanoïque, pentanoïque, hexanoïque ou phtalique.

Pour préparer les produits dans lesquels R représente un radical alkoxy ayant de 1 à 5 atomes de carbone, alkényloxy ayant de 2 à 5 atomes de carbone, cycloalkyloxy ayant de 3 à 7 atomes de carbone ou aralkyloxy ayant de 7 à 12 atomes de carbone, on utilise des chloroformiates correspondants.

On utilise de préférence un dérivé réactionnel d'un acide $R—CO_2H$ dans lequel R représente un radical alkyle ayant de 2 à 5 atomes de carbone ou un radical phényle.

Préférentiellement, le radical R est substitué par un radical carboxyle. Le dérivé réactionnel que l'on fait agir sur l'acide 3α, 7β, 12α-trihydroxycholanique est alors un dérivé des acides malonique, succinique, glutarique, adipique ou phtalique, en particulier l'acide succinique.

Bien entendu, le radical R peut être également substitué par un ou plusieurs autres substituants choisis par exemple parmi les atomes d'halogènes tels que fluor, chlore, brome, iode. On peut également citer par exemple un dérivé réactionnel de l'acide trifluoroacétique.

Le dérivé réactionnel que l'on utilise peut être l'un des dérivés classiques tels qu'un halogénure d'acide (chlorure de préférence), un ester activé ou un amide d'acide.

Parmi les chloroformiates que l'on peut utiliser, on peut citer les chloroformiates d'éthyle, d'isobutyle, d'allyle, de cyclohexyle, de benzyle.

On utilise de préférence un anhydride symétrique, mixte ou cyclique, plus préférentiellement cyclique tel que l'anhydride succinique ou phtalique.

La présente invention a donc pour objet préféré le procédé tel que décrit ci-dessus, caractérisé en ce que le dérivé réactionnel d'un radical acyle de formule :

$$R—CO—$$

que l'on utilise est choisi parmi les anhydrides phtalique, propionique et succinique.

L'agent d'oxydation que l'on utilise pour transformer les produits de formule III en produits de formule IV est de préférence choisi parmi le chlore et le brome en milieu basique, les bichromates de sodium et de potassium dans l'eau, l'anhydride chromique dans l'acide sulfurique ou l'acide acétique, les diméthyl et diéthyl sulfoxydes en présence de dicychlohexylcarbodiimide et d'un acide tel que l'acide phosphorique, l'acide trifluoroacétique, l'acide chlorhydrique ou l'acide sulfurique.

La saponification du produit de formule IV est effectuée dans les conditions habituelles. On peut par exemple utiliser la soude, la potasse ou l'hydroxyde d'ammonium. Cette saponification est suivie éventuellement d'une acidification par l'acide chlorhydrique.

Bien entendu, le produit de formule V peut être isolé sous forme du sel obtenu lors de la saponification, préférentiellement le sel de sodium ou de potassium mais également le sel d'ammonium.

Le produit de formule V peut, si désiré, être purifié sous la forme de sel avant l'acidification. La réduction du produit de formule V en acide ursodésoxycholique est très préférentiellement réalisée par la réaction dite de WOLFF-KISHNER, c'est-à-dire à l'aide d'hydrazine en présence de diéthylène glycol et d'une base, de préférence, la potasse.

On peut également effectuer la réaction de WOLFF-KISHNER à basse température dans le diméthylsulfoxyde en présence de tertbutylate de potassium.

La réduction peut également être effectuée par la méthode dite de Clemmensen en présence d'amalgame de zinc. De même, la réduction pourrait être effectuée par l'hydrogénolyse d'un thiocétal préparé à partir de la cétone.

Parmi les conditions énoncées ci-dessus, on préfère agir l'anhydride succinique sur le produit de formule II, de sorte que le substituant R dans les produits de formules R CO—, III et IV, représente un radical $HO_2CO—CH_2—CH_2$ et que l'agent d'oxydation que l'on fait agir sur le produit de formule III soit le brome en présence de carbonate acide de sodium.

Bien entendu, l'exemple fourni ci-après représente une exécution préférentielle du procédé qui toutefois ne limite pas la portée de la présente invention.

L'invention a donc plus particulièrement pour objet un procédé de préparation de l'acide ursodésoxycholique, caractérisé en ce que l'on traite l'acide 3α, 7β, 12α-trihydroxycholanique par l'anhydride succinique, traite le produit obtenu par le brome en présence de carbonate acide de sodium, traite ensuite le produit résultant par la soude puis par l'acide chlorhydrique et traite enfin le produit obtenu par l'hydrazine en présence de diéthylène glycol et de potasse.

Bien entendu, le produit attendu, c'est-à-dire l'acide ursodésoxycholique, peut être purifié, si désiré, par les méthodes connues dans la littérature. On peut citer, par exemple, des procédés décrits dans les Brevets Japonais 154.864/1975, 154. 866/1975 et 153.954/1977.

La présente invention a également pour objet, à titre de produits industriels nouveaux et notamment à titre de produits industriels nouveaux nécessaires pour la préparation de l'acide ursodésoxycholique, les produits de formule A :

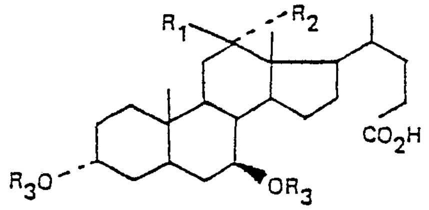

(A)

dans laquelle soit $R_1$ représente un atome d'hydrogène et $R_2$ représente un radical hydroxyle, soit $R_1$ et $R_2$ forment ensemble un radical céto, $R_3$ représente un atome d'hydrogène ou un radical $R-\underset{\underset{O}{\|}}{C}-$, R ayant la signification indiquée ci-dessus, étant entendu que $RR_3$ ne peut pas représenter un atome d'hydrogène lorsque $R_1$ représente un atome d'hydrogène et $R_2$ un radical hydroxyle, ainsi que les sels alcalins ou d'ammonium des produits de formule A, ces produits et leurs sels étant sous forme anhydre ou hydratée.

Les sels alcalins préférés sont les sels de sodium et de potassium. L'invention a plus particulièrement pour objet, à titre de produits industriels nouveaux et notamment à titre de produits industriels nouveaux nécessaires pour la préparation de l'acide ursodésoxycholique, les produits de formule :

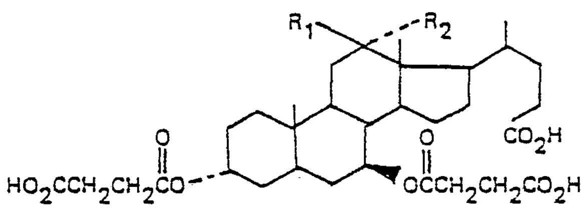

dans laquelle $R_1$ et $R_2$ ont la signification indiquée ci-dessus, ainsi que le produit de formule :

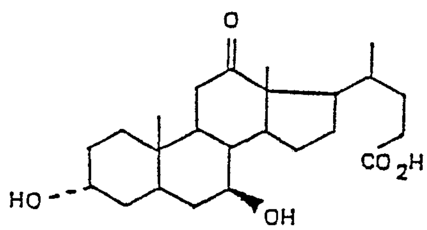

ainsi que ses sels de sodium et d'ammonium, le produit et ses sels étant sous forme anhydre ou hydratée.

L'exemple suivant illustre l'invention sans toutefois la limiter.

Exemple : Acide ursodésoxycholique.

Stade A : Acide 3α, 7β, bis (3-carboxypropanoyloxy)12α-hydroxycholanique.

On agite sous atmosphère d'azote un mélange de 400 g d'acide 3α, 7β, 12β-trihydroxycholanique dans 1 000 $cm^3$ de méthyléthylpyridine puis porte le milieu à 90 °C en 30 minutes. On distille ensuite 200 $cm^3$ de méthyléthylpyridine hydratée sous vide. On refroidit à 50 °C, introduit de l'azote et ajoute dans ces conditions 245 g d'anhydride succinique.

On chauffe ensuite en 50 minutes à 98 °C puis maintient à cette température pendant 3 heures en agitant et sous azote. On refroidit ensuite sous azote à 20 °C.

Stade B : Acide 3α, 7β-bis-(3-carboxypropanoyloxy) 12-cétocholanique.

On ajoute ensuite, sous agitation et atmosphère d'azote, 600 $cm^3$ d'eau déminéralisée et 800 $cm^3$ de tert-butanol maintenu à 25-30 °C au milieu obtenu au stade A.

On ajoute ensuite très progressivement 276 g de carbonate acide de sodium. On réchauffe ensuite à 35 °C, et introduit, en maintenant la température à 35 °C, 276 g de brome. On poursuit ensuite l'agitation 16 heures à la température de 35 °C.

On introduit ensuite une solution préparée extemporanément de 50 g de métabisulfite de sodium dans 100 $cm^3$ d'eau déminéralisée. On maintient 30 minutes sous agitation.

Stade C : Acide 3α, 7β-dihydroxy 12-céto cholanique.

On ajoute, sous atmosphère d'azote, en 30 minutes environ, 1 200 $cm^3$ d'eau déminéralisée puis 700

$cm^3$ de lessive de soude à 30,5 %, au milieu réactionnel obtenu au stade B.

On chauffe alors sous agitation et atmosphère d'azote puis distille à pression ordinaire jusqu'à obtention de vapeurs à + 96 °C.

On recueille environ 1 200 $cm^3$ d'un mélange tert-butanol-eau-méthyléthylpyridine. On poursuit la distillation tout en maintenant le volume réactionnel constant par ajout d'eau déminéralisée, jusqu'à absence de méthyléthylpyridine dans le distillat (on ajoute environ 3 000 $cm^3$ d'eau).

On refroidit régulièrement en 3 heures jusqu'à 20 °C, puis maintient à cette température sous azote pendant 16 heures. On essore le sel de sodium cristallisé et le lave par un mélange de 720 $cm^3$ d'eau déminéralisée et 80 $cm^3$ de lessive de soude à 30,5 %. On obtient environ 700 g de sel de sodium humide du produit recherché.

Le sel de sodium de l'acide 3α,7β-dihydroxy 12-céto cholanique est purifié comme suit : On agite sous atmosphère d'azote et porte au reflux en 30 minutes un mélange obtenu en ajoutant à 700 g de produit brut préparé ci-dessus, 1 440 $cm^3$ d'eau déminéralisée et 160 $cm^3$ de lessive de soude à 30,5 %.

On maintient au reflux 30 minutes, refroidit ensuite jusqu'à 20 °C en 2 heures, agite 2 heures à 20 °C sous azote, puis essore et lave avec un mélange de 720 $cm^3$ d'eau déminéralisée et 80 $cm^3$ de lessive de soude à 30,5 %.

On effectue une seconde purification identique et obtient environ 500 g de sel de sodium du produit recherché. Ces 500 g de sel de sodium du produit recherché sont ajoutés sous agitation à 2 000 $cm^3$ d'eau déminéralisée.

On ajoute ensuite en 30 minutes environ tout en maintenant la température entre 20 et 25 °C, une quantité suffisante d'acide chlorhydrique pour obtenir un pH inférieur ou égal à 2 (soit environ 100 $cm^3$). On agite 2 heures à 20 °C puis essore et lave jusqu'à absence de chlorures dans les eaux de lavages soit avec au total 2 000 $cm^3$ d'eau déminéralisée. On sèche en étuve à 60 °C jusqu'à une teneur en eau de 4,5 % environ et obtient ainsi 246,4 g de produit recherché, soit 235,3 g de produit sec. $[\alpha]_D = + 120,5 \pm 2,5°$ (c = 1 % dans l'éthanol).

Stade D : Acide ursodésoxycholique.

On agite et chauffe à 120 °C sous azote un mélange obtenu en ajoutant à 400 g d'acide 3α,7β-dihydroxy 12-céto cholanique 640 $cm^3$ de diéthylèneglycol (le produit se dissout après 110 °C).

On introduit ensuite, en 15 minutes et en maintenant la température à 120 °C, 160 $cm^3$ de lessive de potasse à 50° Bé. On introduit ensuite sous agitation et balayage d'azote et en maintenant la température à 120 °C, 100 $cm^3$ d'hydrazine hydratée pure. On maintient ensuite le milieu réactionnel sous agitation et sous azote à 120 °C pendant 2 heures.

On chauffe ensuite le milieu réactionnel sous agitation et sous azote en 2 heures jusqu'à obtention d'une température, de 196 °C dans le milieu. (Au cours de la montée en température, on distille l'eau de déshydratation et l'hydrazine en excès. On récupère environ 215 $cm^3$ d'un mélange eau-hydrazine).

On maintient le milieu sous agitation et sous azote pendant 4 h 30 à une température comprise entre 195 et 197 °C. On refroidit le milieu réactionnel à 50 °C sous agitation et balayage d'azote et obtient une solution potassique d'acide ursodésoxycholique (environ 800 $cm^3$).

A un mélange de 800 $cm^3$ d'eau déminéralisée et 300 $cm^3$ d'acide chlorhydrique, on introduit la solution potassique d'acide ursodésoxycholique précédente, sous agitation et balayage d'azote en 30 minutes et en maintenant la température à 50 °C. On refroidit sous azote à 27 °C, ajuste le pH à 1 par addition d'acide chlorhydrique. On maintient la suspension pendant 3 heures à 27 °C, essore et lave à l'eau jusqu'à absence de chlorures dans les eaux de lavage (soit avec 4 000 $cm^3$ d'eau déminéralisée en tout).

On sèche le produit obtenu en étuve à 60 °C.

On obtient 378,5 g d'acide ursodésoxycholique.

**Revendications** (pour les Etats contractants : AT, BE, CH, DE, GB, LI, NL)

1. Procédé de préparation de l'acide ursodésoxycholique de formule I

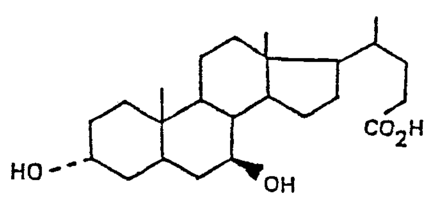

(I)

caractérisé en ce que l'on traite l'acide 3α,7α,12α-trihydroxycholanique de formule II OH

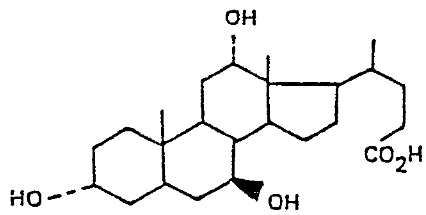

(II)

par un dérivé réactionnel d'un radical organique de formule

R—CO—

dans laquelle R représente un atome d'hydrogène ou un radical alkyle ou alkoxy ayant de 1 à 5 atomes de carbone ou alkényloxy ayant de 2 à 5 atomes de carbone, un radical cycloalkyloxy ayant de 3 à 7 atomes de carbone ou aralkyloxy ayant de 7 à 12 atomes de carbone, un radical aryle ou aralkyle ayant de 6 à 12 atomes de carbone, chacun de ces radicaux portant éventuellement un ou plusieurs substituants, pour obtenir un produit de formule III :

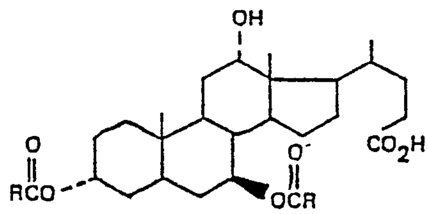

(III)

produit de formule III que l'on traite par le brome en milieu basique pour obtenir un produit de formule IV

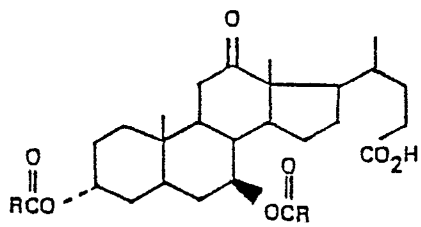

(IV)

produit de formule IV que l'on saponifie pour obtenir un produit de formule V :

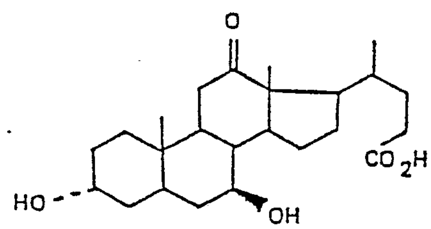

(V)

ou un sel de ce produit, produit de formule V ou sel que l'on traite par l'hydrazine en présence de diéthylène glycol et d'une base pour obtenir l'acide ursodésoxycholique cherché.

2. Procédé selon la revendication 1 de préparation de l'acide ursodésoxycholique, caractérisé en ce que le dérivé réactionnel d'un radical acyle de formule

R—CO—

que l'on utilise est choisi parmi les anhydrides phtalique, propionique et succinique.

3. Procédé selon la revendication 1, caractérisé en ce que la saponification du produit de formule IV est effectuée par une base choisie parmi la soude, la potasse et l'hydroxyde d'ammonium et est suivie d'une acidification par l'acide chlorhydrique.

4. Procédé selon les revendications 1 et 2 de préparation de l'acide ursodésoxycholique, caractérisé en ce que l'on fait agir l'anhydride succinique sur le produit de formule II pour obtenir un produit de formule III dans laquelle R représente un radical $HO_2C—CH_2—CH_2—$.

5. Procédé selon la revendication 1 de préparation de l'acide ursodésoxycholique, caractérisé en ce que l'on fait agir le brome sur le produit de formule III en présence de carbonate acide de sodium.

6. Procédé selon l'une des revendications 1 à 5, de préparation de l'acide ursodésoxycholique, caractérisé en ce que l'on traite l'acide 3α,7β,12α-trihydroxycholanique par l'anhydride succinique, traite

le produit obtenu par le brome en présence de carbonate acide de sodium, traite ensuite le produit résultant par la soude puis par l'acide chlorhydrique et traite enfin le produit obtenu par l'hydrazine en présence de diéthylèneglycol et de potasse.

**Revendications** (pour l'Etat contractant IT)

1. Procédé de préparation de l'acide ursodésoxycholique de formule I

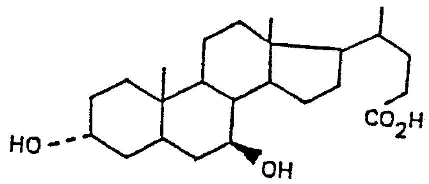

(I)

caractérisé en ce que l'on traite l'acide 3α,7β,12α-trihydroxycholanique de formule II

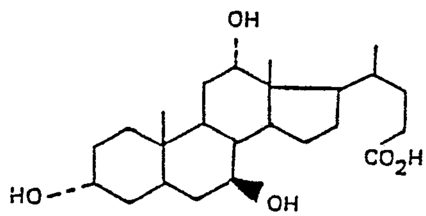

(II)

par un dérivé réactionnel d'un radical organique de formule

R—CO—

dans laquelle R représente un atome d'hydrogène ou un radical alkyle ou alkoxy ayant de 1 à 5 atomes de carbone ou alkényloxy ayant de 2 à 5 atomes de carbone, un radical cycloalkyloxy ayant de 3 à 7 atomes de carbone ou aralkyloxy ayant de 7 à 12 atomes de carbone, un radical aryle ou aralkyle ayant de 6 à 12 atomes de carbone, chacun de ces radicaux portant éventuellement un ou plusieurs substituants, pour obtenir un produit de formule III :

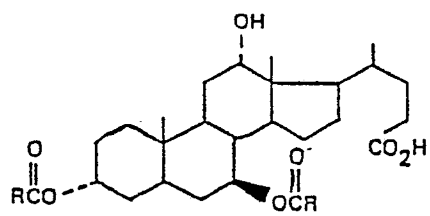

(III)

produit de formule III que l'on traite par un agent d'oxydation pour obtenir un produit de formule IV

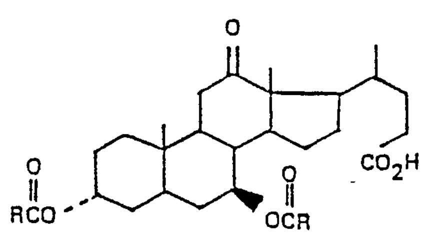

(IV)

produit de formule IV que l'on saponifie pour obtenir un produit de formule V :

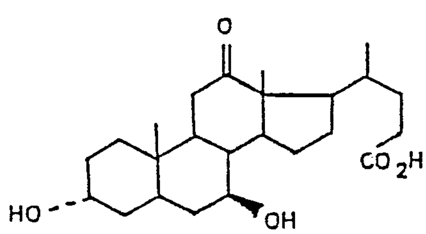

(V)

ou un sel de ce produit, produit de formule V ou sel que l'on traite par un agent de réduction pour obtenir l'acide ursodésoxycholique cherché.

2. Procédé selon la revendication 1 de préparation de l'acide ursodésoxychiolique, caractérisé en ce que le dérivé réactionnel d'un radical acyle de formule

$$R—CO—$$

que l'on utilise est choisi parmi les anhydrides phtalique propionique et succinique.

3. Procédé selon la revendication 1, caractérisé en ce que l'agent d'oxydation que l'on utilise pour transformer un produit de formule III en un produit de formule IV est choisi parmi le chlore et le brome en milieu basique.

4. Procédé selon la revendication 1, caractérisé en ce que la saponification du produit de formule IV est effectuée par une base choisie parmi la soude, la potasse et l'hydroxyde d'ammonium et est suivie d'une acidification par l'acide chlorhydrique.

5. Procédé selon la revendication 1, caractérisé en ce que la transformation du produit de formule V en acide ursodésoxycholique est effectuée par l'hydrazine en présence de diéthylène glycol et d'une base.

6. Procédé selon les revendications 1 et 2 de préparation de l'acide ursodésoxycholique, caractérisé en ce que l'on fait agir l'anhydride succinique sur le produit de formule II pour obtenir un produit de formule III dans laquelle R représente un radical $HO_2C—CH_2—CH_2—$.

7. Procédé selon les revendications 1 et 3 de préparation de l'acide ursodésoxycholique, caractérisé en ce que l'agent d'oxydation que l'on fait agir sur le produit de formule III est le brome en présence de carbonate acide de sodium.

8. Procédé selon l'une des revendications 1 à 7, de préparation de l'acide ursodésoxycholique, caractérisé en ce que l'on traite l'acide 3α,7β,12α-trihydroxycholanique par l'anhydride succinique, traite le produit obtenu par le brome en présence de carbonate acide de sodium, traite ensuite le produit résultant par la soude puis par l'acide chlorhydrique et traite enfin le produit obtenu par l'hydrazine en présence de diéthylèneglycol et de potasse.

9. A titre de produits industriels nouveaux, les produits de formule A :

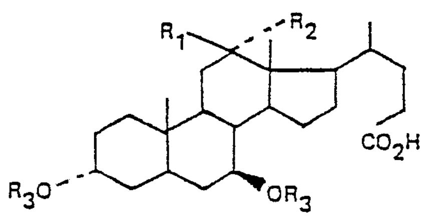

(A)

dans laquelle soit $R_1$ représente un atome d'hydrogène et $R_2$ représente un radical hydroxyle, soit $R_1$ et $R_2$ forment ensemble un radical céto, $R_3$ représente un atome d'hydrogène ou un radical $R-\underset{\underset{O}{\|}}{C}-$, R ayant la signification indiquée à la revendication 1, étant entendu que $R_3$ ne peut pas représenter un atome d'hydrogène lorsque $R_1$ représente un atome d'hydrogène et $R_2$ un radical hydroxyle, ainsi que les sels alcalins ou d'ammonium des produits de formule A, ces produits et leurs sels étant sous forme anhydre ou hydratée.

10. A titre de produits industriels nouveaux selon la revendication 9, les produits de formule :

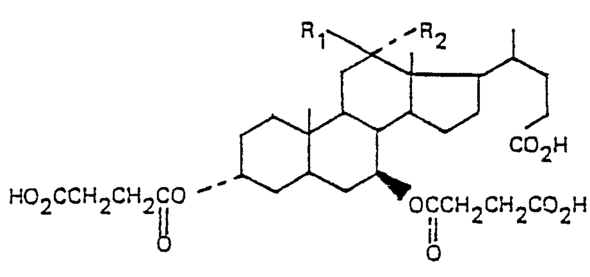

dans laquelle $R_1$ et $R_2$ ont la signification indiquée à la revendication 9.

11. A titre de produit industriel nouveau selon la revendication 9, le produit de formule :

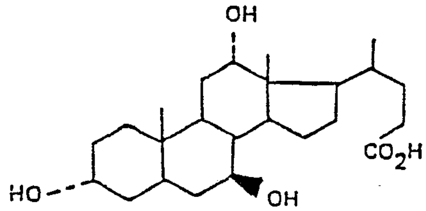

ainsi que ses sels de sodium et d'ammonium, le produit et ses sels étant sous forme anhydre ou hydratée.

**Claims** (for the Contracting States : AT, BE, CH, DE, GB, LI, NL)

1. Preparation process of ursodesoxycholic acid with the formula I

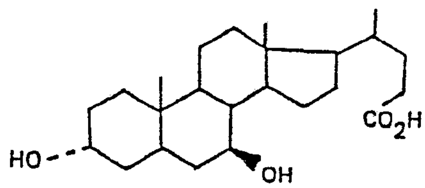

(I)

characterized in that 3α, 7β, 12α-trihydroxycholanic acid with the formula II

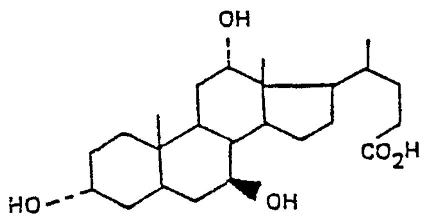

(II)

is treated with a reactional derivative of an organic radical with the formula

R—CO—

in which R represents a hydrogen atom or an alkyl or alkoxy radical having from 1 to 5 carbon atoms or an alkenyloxy radical having from 2 to 5 carbon atoms, a cycloalkyloxy radical having from 3 to 7 carbon atoms or an aralkyloxy radical having from 7 to 12 carbon atoms, an aryl or aralkyl radical having from 6 to 12 carbon atoms, each of these radicals possibly carrying one or more substituents, so as to obtain a product with the formula III :

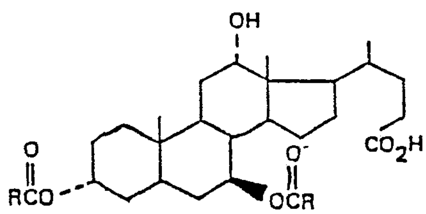

(III)

which product with the formula III is treated with bromine in a basic medium so as to obtain a product with the formula IV

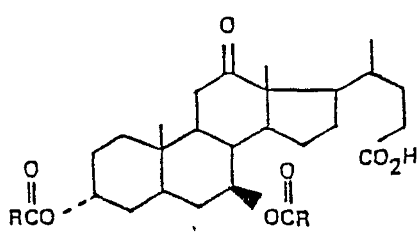

(IV)

which product with the formula IV is saponified so as to obtain a product with the formula V :

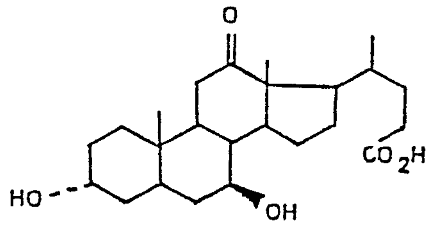

(V)

or a salt of this product, which product with the formula V or which salt is treated with hydrazine in the presence of diethylene glycol and a base so as to obtain the sought ursodesoxycholic acid.

2. Process according to claim 1 for the preparation of ursodesoxycholic acid, characterized in that the reactional derivative of an acyl radical with the formula

R—CO—

which is used is chosen from phthalic, propionic and succinic anhydrides.

3. Process according to claim 1, characterized in that the saponification of the product with the formula IV is carried out by a base chosen from sodium, potassium and ammonium hydroxide and is followed by acidification with hydrochloric acid.

4. Process according to claims 1 and 2 for the preparation of ursodesoxycholic acid, characterized in that succinic anhydride is made to react on the product with the formula II so as to obtain a product with the formula III in which R represents a radical $HO_2C—CH_2—CH_2—$.

5. Process according to claim 1 for the preparation of ursodesoxycholic acid, characterized in that bromine is made to act on the product with the formula III in the presence of sodium acid carbonate.

6. Process according to any one of the claims 1 to 5, for the preparation of ursodesoxycholic acid, characterized in that 3α, 7β, 12α-trihydroxycholanic acid is treated with succinic anhydride, the product obtained is treated with bromine in the presence of sodium acid carbonate, the resulting product is then treated with sodium hydroxide then with hydrochloric acid and finally the product obtained is treated with hydrazine in the presence of diethylene glycol and potassium hydroxide.

**Claims** (for the Contracting State IT)

1. Preparation process of ursodesoxycholic acid with the formula I

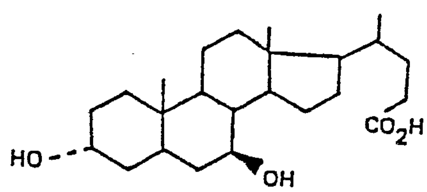

(I)

characterized in that 3α, 7β, 12α-trihydroxycholanic acid with the formula II

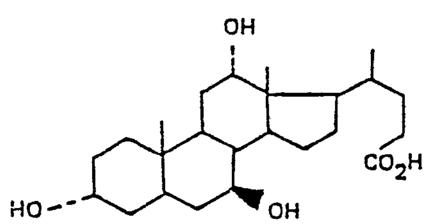

(II)

is treated with a reactional derivative of an organic radical with the formula

R—CO—

in which R represents a hydrogen atom or an alkyl or alkoxy radical having from 1 to 5 carbon atoms or an alkenyloxy radical having from 2 to 5 carbon atoms, a cycloalkyloxy radical having from 3 to 7 carbon atoms or an aralkyloxy radical having from 7 to 12 carbon atoms, an aryl or aralkyl radical having from 6 to 12 carbon atoms, each of these radicals possibly carrying one or more substituents, so as to obtain a product with the formula III :

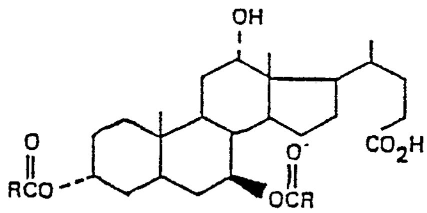

(III)

which product with the formula III is treated with an oxidizing agent so as to obtain a product with the formula IV

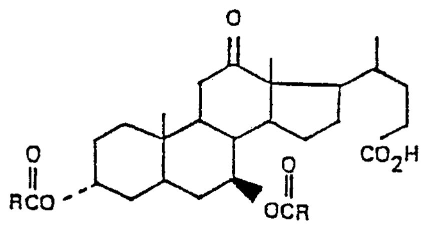

(IV)

which product with the formula IV is saponified so as to obtain a product with the formula V :

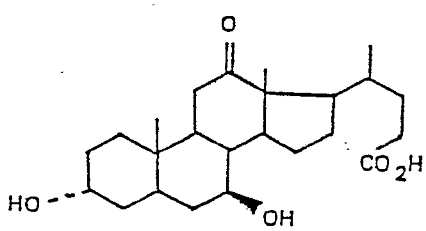

(V)

or a salt of this product, which product with the formula V or which salt is treated with a reducing agent so as to obtain the sought ursodesoxycholic acid.

2. Process according to claim 1 for the preparation of ursodesoxycholic acid, characterized in that the reactional derivative of an acyl radical with the formula

R—CO—

which is used is chosen from phthalic, propionic and succinic anhydrides.

3. Process according to claim 1, characterized in that the oxidizing agent which is used to convert a product with the formula III into a product with the formula IV is chosen from chlorine and bromine in a basic medium.

4. Process according to claim 1, characterized in that the saponification of the product with the formula IV is carried out by a base chosen from sodium, potassium and ammonium hydroxide and is followed by acidification with hydrochloric acid.

5. Process according to claim 1, characterized in that the conversion of the product with the formula V into ursodesoxycholic acid is carried out by hydrazine in the presence of diethylene glycol and a base.

6. Process according to claims 1 and 2 for the preparation of ursodesoxycholic acid, characterized in that succinic anhydride is made to react on the product with the formula II so as to obtain a product with the formula III in which R represents a radical $HO_2C—CH_2—CH_2—$.

7. Process according to claims 1 and 3 for the preparation of ursodesoxycholic acid, characterized in that the oxidizing agent which is made to act on the product with the formula III is bromine in the presence of sodium acid carbonate.

8. Process according to any one of the claims 1 to 7, for the preparation of ursodesoxycholic acid, characterized in that 3α, 7β, 12α-trihydroxycholanic acid is treated with succinic anhydride, the product obtained is treated with bromine in the presence of sodium acid carbonate, the resulting product is then treated with sodium hydroxide then with hydrochloric acid and finally the product obtained is treated with hydrazine in the presence of diethylene glycol and potassium hydroxide.

9. As new industrial products, products with the formula A :

(Voir cliché page 12)

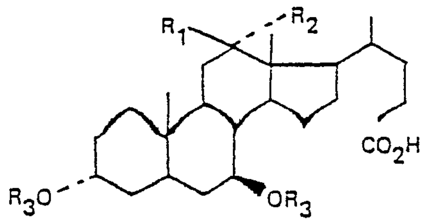

(A)

in which either $R_1$ represents a hydrogen atom and $R_2$ represents a hydroxyl radical, or $R_1$ and $R_2$ together form a keto radical, $R_3$ represents a hydrogen atom or a radical $R-\underset{\underset{O}{\|}}{C}-$,

R having the significance indicated in Claim 1, it being understood that $R_3$ can not represent a hydrogen atom when $R_1$ represents a hydrogen atom and $R_2$ a hydroxyl radical, as well as the alkaline or ammonium salts of the products with the formula A, these products and their salts being in an anhydrous or hydrated form.

10. As new industrial products according to claim 9, the products with the formula :

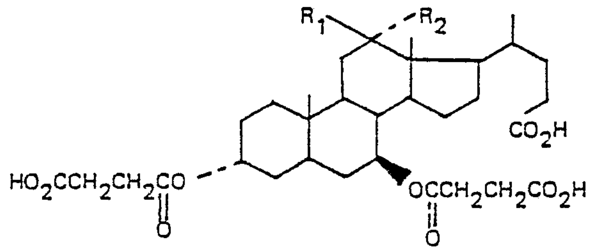

in which $R_1$ and $R_2$ have the significance indicated in claim 9.

11. As new industrial product according to claim 9, the product with the formula :

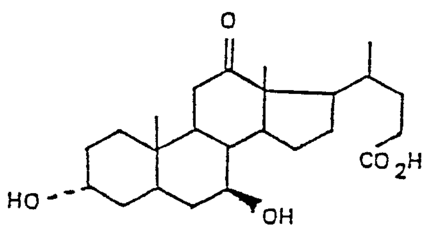

as well as its salts of sodium and ammonium, the product and its salts being in an anhydrous or hydrated form.

**Patentansprüche** (für die Vertragsstaaten : AT, BE, CH, DE, GB, LI, NL)

1. Verfahren zur Herstellung der Ursodesoxycholsäure der Formel I

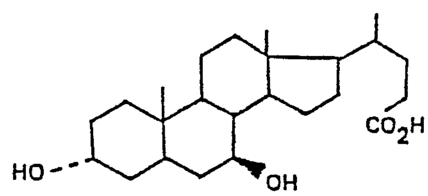

(I)

dadurch gekennzeichnet, daß man die 3α, 7β, 12α-Trihydroxycholansäure der Formel II

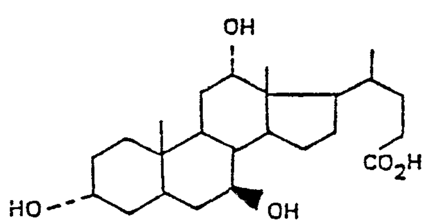

(II)

mit einem reaktiven Derivat eines organischen Restes der Formel

R—CO—

worin R ein Wasserstoffatom oder einen Alkyl- oder Alkoxyrest mit 1 bis 5 Kohlenstoffatomen oder einen Alkenyloxyrest mit 2 bis 5 Kohlenstoffatomen, einen Cycloalkoxyrest mit 3 bis 7 Kohlenstoffatomen oder einen Aralkoxyrest mit 7 bis 12 Kohlenstoffatomen, einen Aryl- oder Aralkylrest mit 6 bis 12 Kohlenstoffatomen bedeutet, wobei ein jeder dieser Reste gegebenenfalls einen oder mehrere Substituenten enthält, behandelt, um ein Produkt der Formel III

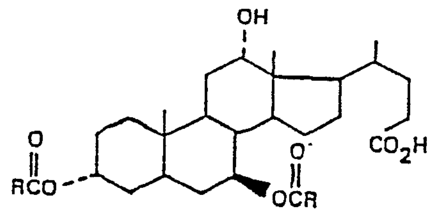

(III)

zu erhalten, welches Produkt der Formel III man mit Brom in basischem Milieu behandelt, um ein Produkt der Formel IV

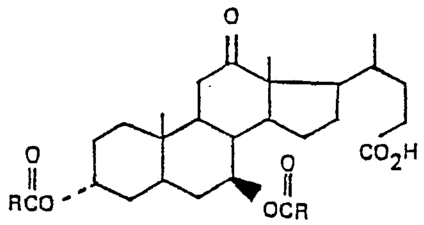

(IV)

zu erhalten, welches Produkt der Formel IV man verseift, um ein Produkt der Formel V

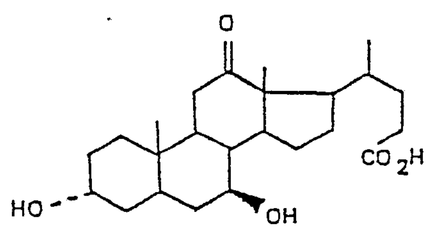

(V)

oder ein Salz dieses Produktes zu erhalten, welches Produkt der Formel V oder welches Salz man mit Hydrazin in Gegenwart von Diethylenglykol und einer Base behandelt, um die gewünschte Ursodesoxycholsäure zu erhalten.

2. Verfahren gemäß Anspruch 1 zur Herstellung der Ursodesoxychlosäure, dadurch gekennzeichnet, daß das reaktive Derivat eines Acylrestes der Formel

R—CO—

das man verwendet, unter den Phthalsäure-, Propionsäure- und Bernsteinsäureanhydriden ausgewählt wird.

3. Verfahren gemäß Anspruch 1, dadurch gekennzeichnet, daß die Verseifung des Produktes der Formel IV mit einer Base, ausgewählt unter Natronlauge, Kalilauge und Ammoniumhydroxid, durchgeführt wird und sich ein Ansäuern mit Chlorwasserstoffsäure anschließt.

4. Verfahren gemäß den Ansprüchen 1 und 2 zur Herstellung von Ursodesoxycholsäure, dadurch gekennzeichnet, daß man Bernsteinsäureanhydrid mit dem Produkt der Formel II umsetzt, um ein Produkt der Formel III zu erhalten, worin R einen Rest $HO_2C—CH_2—CH_2—$ bedeutet.

5. Verfahren gemäß Anspruch 1 zur Herstellung der Ursodesoxycholsäure, dadurch gekennzeichnet, daß man Brom mit dem Produkt der Formel III in Gegenwart von Natriumbicarbonat umsetzt.

6. Verfahren gemäß einem der Ansprüche 1 bis 5 zur Herstellung der Ursodesoxycholsäure, dadurch gekennzeichnet, daß man die 3α, 7β, 12α-Trihydroxycholansäure mit Bernsteinsäureanhydrid behandelt, das erhaltene Produkt mit Brom in Gegenwart von Natriumbicarbonat behandelt, hierauf das entstandene Produkt mit Natronlauge und dann mit Chlorwasserstoffsäure behandelt und schließlich das erhaltene Produkt mit Hydrazin in Gegenwart von Diethylenglykol und Kalilauge behandelt.

**Patentansprüche** (für den Vertragsstaat IT)

1. Verfahren zur Herstellung der Ursodesoxycholsäure der Formel I

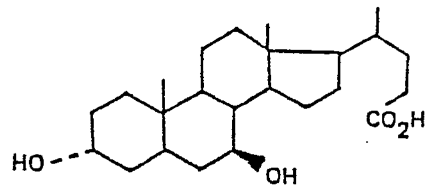

(I)

dadurch gekennzeichnet, daß man die 3α, 7β, 12α-Trihydroxycholansäure der Formel II

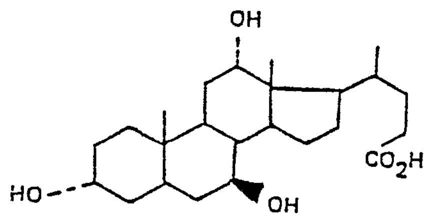

(II)

mit einem reaktiven Derivat eines organischen Restes der Formel

R—CO—

worin R ein Wasserstoffatom oder einen Alkyl- oder Alkoxyrest mit 1 bis 5 Kohlenstoffatomen oder einen Alkenyloxyrest mit 2 bis 5 Kohlenstoffatomen, einen Cycloalkoxyrest mit 3 bis 7 Kohlenstoffatomen oder einen Aralkoxyrest mit 7 bis 12 Kohlenstoffatomen bedeutet, wobei ein jeder dieser Reste gegebenenfalls einen oder mehrere Substituenten enthält, behandelt, um ein Produkt der Formel III

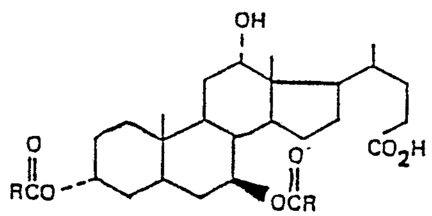

(III)

zu erhalten, welches Produkt der Formel III man mit einem Oxidationsmittel behandelt, um ein Produkt der Formel IV

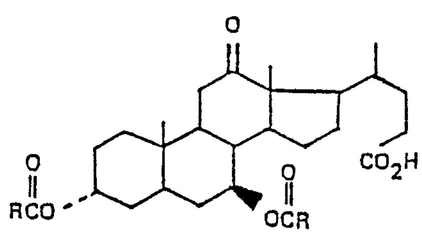

(IV)

zu erhalten, welches Produkt der Formel IV man verseift, um ein Produkt der Formel V

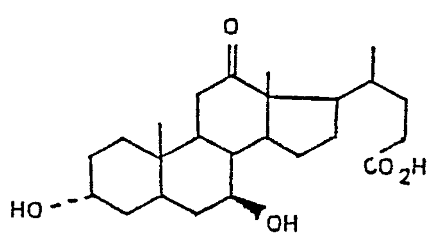

(V)

oder ein Salz dieses Produktes zu erhalten, welches Produkt der Formel V oder Salz man mit einem Reduktionsmittel behandelt, um die gewünschte Ursodesoxycholsäure zu erhalten.

2. Verfahren gemäß Anspruch 1 zur Herstellung der Ursodesoxycholsäure, dadurch gekennzeichnet, daß das reaktive Derivat eines Acylrestes der Formel

$$R—CO—$$

das man verwendet, unter den Phthalsäure-, Propionsäure- und Bernsteinsäureanhydriden ausgewählt wird.

3. Verfahren gemäß Anspruch 1, dadurch gekennzeichnet, daß das Oxidationsmittel, das man für die Überführung eines Produktes der Formel III in ein Produkt der Formel IV verwendet, unter Chlor und Brom in basischem Milieu ausgewählt wird.

4. Verfahren gemäß Anspruch 1, dadurch gekennzeichnet, daß die Verseifung des Produktes der Formel IV mit einer Base, ausgewählt unter Natronlauge, Kalilauge und Ammoniumhydroxid, durchgeführt wird und sich ein Ansäuern mit Chlorwasserstoffsäure anschließt.

5. Verfahren gemäß Anspruch 1, dadurch gekennzeichnet, daß die Überführung des Produktes der Formel V in die Ursodesoxycholsäure mit Hydrazin in Gegenwart von Diethylenglykol und einer Base erfolgt.

6. Verfahren gemäß den Ansprüchen 1 und 2 zur Herstellung der Ursodesoxycholsäure, dadurch gekennzeichnet, daß man Bernsteinsäureanhydrid mit dem Produkt der Formel II umsetzt, um ein Produkt der Formel III zu erhalten, worin R einen Rest $HO_2C—CH_2—CH_2—$ bedeutet.

7. Verfahren gemäß einem der Ansprüche 1 und 3 zur Herstellung von Ursodesoxycholsäure, dadurch gekennzeichnet, daß das Oxidationsmittel, das man mit dem Produkt der Formel III umsetzt, Brom in Gegenwart von Natriumbicarbonat ist.

8. Verfahren gemäß einem der Ansprüche 1 bis 7 zur Herstellung von Ursodesoxycholsäure, dadurch gekennzeichnet, daß man 3α, 7β, 12α-Trihydroxycholansäure mit Bernsteinsäureanhydrid behandelt, das erhaltene Produkt mit Brom in Gegenwart von Natriumbicarbonat behandelt, hierauf das entstandene Produkt mit Natronlauge und dann mit Chlorwasserstoffsäure behandelt und schließlich das erhaltene Produkt mit Hydrazin in Gegenwart von Diethylenglykol und Kalilauge behandelt.

9. Als neue, industrielle Produkte die Produkte der Formel A

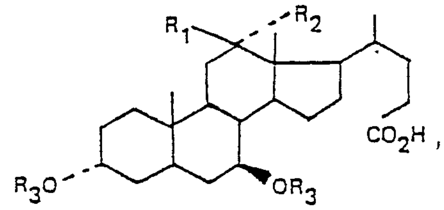

(A)

worin entweder $R_1$ ein Wasserstoffatom bedeutet und $R_2$ einen Hydroxylrest bedeutet oder $R_1$ und $R_2$ gemeinsam einen Ketorest bilden, $R_3$ ein Wasserstoffatom oder einen Rest $R-\underset{O}{\underset{\|}{C}}-$ bedeutet, wobei R die in Anspruch 1 angegebene Bedeutung besitzt, wobei $R_3$ kein Wasserstoffatom bedeuten kann, wenn $R_1$ ein Wasserstoffatom bedeutet und $R_2$ einen Hydroxylrest bedeutet, sowie die Alkali- und Ammoniumsalze der Produkte der Formel A, wobei diese Produkte und deren Salze in wasserfreier Form oder in Hydratform vorliegen können.

10. Als neue, industrielle Produkte gemäß Anspruch 9 die Produkte der Formel

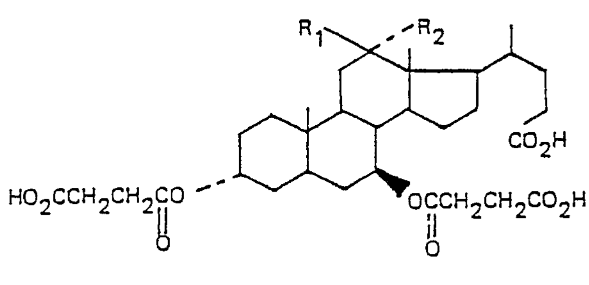

worin $R_1$ und $R_2$ die in Anspruch 9 angegebene Bedeutung besitzen.

11. Als neues, industrielles Produkt gemäß Anspruch 9 das Produkt der Formel

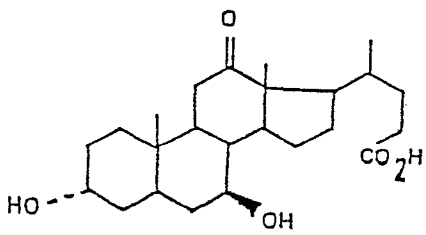

sowie seine Natrium- und Ammoniumsalze, wobei das Produkt und seine Salze in wasserfreier oder in Hydratform vorliegen können.